# EUROPEAN PATENT APPLICATION

(11) **EP 2 450 432 A1**
(43) Date of publication of application: **09.05.2012**
(21) Application number: 11169669.6
(22) Date of filing: 13.06.2011
(51) Int. Cl.: C12N 5/0797, C12N 5/0775

(54) **Materials composition and methods to control neural progenitor and stem cell attachment, proliferation and guide cell differentiation**

(30) Priority: 05.11.2010 EP 10190255
(71) Applicant: Beijing Allgens Medical Science & Technology Co., Ltd., Beijing 100085 (CN)
(72) Inventor: Hu, Eric G., New Jersey, NJ 07920 (US); Hu, Kun, 100085 Haidian District (CN); Ren, Yongjuan, 100085 Haidian District (CN)
(74) Representative: Clegg, Richard Ian

(57) **Abstract**

This invention disclosure provides culture surface compositions and methods that control the NPCs and NSCs, and/or MSCs to either largely maintain their phenotypes, or differentiate on these culture surfaces in guided lineage. The culture surface composition can be constructed to contain either OH functional groups, or ―NH₂ groups to control the adherence, migration, proliferation and differentiation of MSCs, providing a methods to direct NSCs lineage specification in neural tissue engineering for a wide variety of neurological diseases.

## Description

### REFERENCES

### PUBLICATIONS

1. Fibroblast growth factor 2(FGF-2) promotes acquisition of epidermal growth factor (EGF) responsiveness in mouse striatal precursor cells: identification of neural precursors responding to both EGF and PFG-2. J Neurosci 1998: 18: 7869-80
2. Engineering substrate topography at the micro- and Nanoscale to control cell function. Christopher J Bettinger, Robert Langer and Jeffrey Borenstein. Angew. Chem. Int. Ed. 2009, 48, 5406-5415
3. The development of high-throughput screening approaches for stem cell engineering. Ying Mei, Michael Goldberg and Daniel Anderson. Current. Opinion in Chem. Bio. 2007, 11: 388-393
4. In vitro behavior of neural stem cells in response to different chemical functional groups. Yong-juan Ren, Han zhang, et al . Bimaterials, (2009) 1036-1044

### FIELD OF THE INVENTION

The present invention relates generally to using materials' surface compositions exhibiting different chemical functional groups to control attachment, proliferation and guide differentiation of p/luripotent stem cells and neural progenitor cells. More specifically, the invention provides compositions and methods for in vitro controlling the attachment, proliferation and differentiation of stem and neural progenitor cells. The stem cells and neural progenitor cells can be useful for therapeutic, diagnostic, drug screening and cytoxicity test.

### BACKGROUND OF THE INVENTION

Description of the Related Art

Disorders of the central nervous system (CNS) include a number and variety of conditions, such as neurodegenerative diseases (e.g. Alzheimer's and Parkinson's), acute brain injury (e.g. stroke, head trauma, cerebral palsy) and neurological dysfunction (e.g. depression, epilepsy, schizophrenia). These neurodegenerative diseases, which include Alzheimer's disease (AD), multiple sclerosis (MS), Huntington's disease (HD), amyotrophic lateral sclerosis (ALS), and Parkinson's disease (PD), have been linked to the degeneration of neural cells in identified locations of the CNS, resulting in an inability of these cells or the relevant brain region to carry out their intended function. Over 1.5 million people in the United States suffer from Parkinson's disease (PD). Once pharmacological treatment for PD is exhausted, patient can only turn to surgical interventions. Current interventions focus on containing PD symptoms, but it is imperative to attempt to reverse the damage of the disease. Such restoration may be possible through transplantation of neuronal progenitor cells.

Grafting of neuronal progenitor cells offers a therapeutic approach to demyelinating diseases, such as multiple sclerosis (MS). In both human demyelinating diseases and rodent models there is substantial evidence that demyelinated neurons are capable of remyelination in vivo. In MS, for example, it appears that there are often cycles of de- and remyelination. Exogenously applied cells have been shown to be capable of remyelinating demyelinated axons in a number of experimental conditions. Success has been shown using dissociated glial cell suspensions prepared from spinal cords, cultures from dissociated brain tissue; oligodendrocyte precursor cells; 0-2A cells and immortalized 0-2A cell lines . 0-2A cells are glial progenitor cells which give rise in vitro only to oligodendrocytes and type II astrocytes. Cells immunopositive in vivo for the 0-2A phenotype have been shown to successfully remyelinate demyelinated neurons in vivo. Injection of a large number of 0-2A cells is required to adequately remyelinate all targeted neurons in vivo. Although 0-2A progenitor cells can be grown in culture, they are capable of only a limited number of divisions. In addition, the isolation technique employs a low yield source (optic nerve) and requires a number of purification steps.

While attempts have been made to propagate neural progenitor cells for use in neurotransplantation and for drug screening, these efforts have met with limited success. The major challenge is the lack of methods to effectively control stem cell growth and differentiation. It has been attempted using various chemicals/growth factors in the culture medium to guide cell differentiation, such as epidermal growth factor (EGF) and basic fibroblast growth factor (bFGF) ¹⁾, however, these attempts also met with limited success. In addition, only a small fraction of stem cells survive when implanted, so there is a need to develop new systems or synthetic microenvironments that encourage survival and integration of neural stem cells or precursors into diseased or injured tissues of the CNS. Researchers have used various materials surface topography ^{2,3)}, chemical functional groups grafted to material surfaces to guide stem cell differentiation ⁴⁾, and our invention disclosed an approach and method to effectively control stem cell attachment, proliferation and differentiation via materials surface properties manipulations.

The present invention discloses surface compositions that can control NSC and neural progenitor cells' attachment, proliferation and differentiation. This surface makes culture of the NSCs and differentiated neural progenitor cells practical for clinical applications such as transplantation or for drug screening.

### SUMMARY OF THE INVENTION

The invention provides composition of the culture surface and methods for the culturing, propagation and manipulation of neural progenitor cells (NPC) and pluripotent stem cells (PSC). The invention provides a culture surface and a culture medium, wherein the hydroxyl concentration of the surface determines the surface hydrophilicity and its surface contact angles could range from 5° to 35°. This culture surface can inhibit NPC and PSC differentiation and maintain these cells in proliferation state. This invention also provides culture surfaces where amino group will promote neuronal differentiation into neurons, astrocytes and oligodendrocytes. Also provided is a cell culture system comprising NPC or PSC, or other Stem Cells (SCs) cultured on these surface that can be successfully maintained for a long period of time.

### BRIEF DESCRIPTION OF THE DRAWINGS

Fig.1. depicts water contact angles for -OH and ―NH₂ modified surfaces.

Fig.2. demonstrates immunochemical staining plot for single cell after cultured for 1 day in contact with -OH and ―NH₂ modified surfaces .

Fig.3 shows fluorescent photomicrographs representing differentiated cell phenotypes from embryonic cerebral cortical neurospheres at 350 neurospheres/cm² in contact with -OH and ―NH₂ modified surfaces under serum-free conditions after cultured for 5 days.

Fig.4 are SEM images of neural stem cells cultured for 1 day on -OH and ―NH₂ modified surfaces at 5000 cells/cm² under serum-free conditions

Fig.5 depicts neurospheres cultured for 1 day on -OH and ―NH₂ modified surfaces at 350 neurospheres/cm² under serum-free condition

Fig.6 illustrates neurospheres cultured for 5 days on -OH and ―NH₂ modified surfaces at 350 neurospheres/cm² under serum-free condition

Fig. 7 MTT reduction activity of neurospheres cultured on -OH and ―NH₂ modified surfaces at day 1, day 3 and day 5.

Fig. 8 MTT reduction activity of single cell cultured on -OH and ―NH₂ modified surfaces at day 1, day 3 and day 5.

Fig.9 Scanning confocal photomicrographs of neural stem cells attached to surfaces with -OH and ―NH₂ groups after incubation for 12 hours.

Fig.10 Scanning confocal photomicrographs of neural stem cells attached to surfaces with different terminal groups after incubation for 5 days.

Fig. 11 Scanning confocal photomicrographs of adipose-derived stem cells attached to surfaces with different terminal groups after incubation for 7 days.

Fig. 12 Optical absorbance of supernatants of adipose-derived stem cells cultured on different terminal chemical groups after incubation with CCK-8 for 4 hours at day 2, day 4 and day 6.

### DETAILED DESCRIPTION OF THE EMBODIMENTS

The present invention disclosed a culture surface and medium which enable effective control over the attachment, proliferation and differentiation of human neural progenitor cells (NPC) and neural stem cell (NSC) and other type of pluripotent stem cells (PSCs). In addition, the invention provides capability of varying the culture surface and medium that allow for manipulation of the cultured NPC to achieve desirable attachment and differentiation. NPC and PSC cultured under our conditions can provide restoration of brain structure and function in an animal model of Parkinson's disease. Moreover, the same culture conditions used to propagate NPC are also applicable for cultivating PSC.

Definitions

All scientific and technical terms used in this application have meanings commonly used in the art unless otherwise specified. As used in this application, the following words or phrases have the meanings specified.

As used herein, "neural progenitor cell" (NPC) refers to cells that are immunopositive for nestin, capable of continuous growth in suspension cultures and, upon exposure to appropriate conditions, can differentiate into neurons or glial cells. A neural progenitor cell, as referred to herein, is capable of surviving for at least 2-3 years in vitro.

As used herein, "pluripotent stem cell" (PSC) refers to cells that are immunopositive for the stem cell marker, Oct4.

As used herein, "genetically modified" refers to cells that have been manipulated to contain a transgene by natural or recombinant methods. For example, NPC or their progeny can be genetically modified by introducing a nucleic acid molecule that encodes a desired polypeptide.

As used herein, "transgene" means DNA that is inserted into a cell and that encodes an amino acid sequence corresponding to a functional protein. Typically, the encoded protein is capable of exerting a therapeutic or regulatory effect on cells of the CNS.

As used herein, "protein" or "polypeptide" includes proteins, functional fragments of proteins, and peptides, whether isolated from natural sources, produced by recombinant techniques or chemically synthesized. Polypeptides of the invention typically comprise at least about 6 amino acids, and are sufficiently long to exert a biological or therapeutic effect.

As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

The term "nucleic acid" or "polynucleotide" refers to a deoxyribonucleotide or ribonucleotide polymer in either single- or double-stranded form, and unless otherwise limited, encompasses known analogs of natural nucleotides that hybridize to nucleic acids in a manner similar to naturally-occurring nucleotides.

As used herein, "pharmaceutically acceptable formulation" includes any material which, when combined with an active ingredient, allows the ingredient to retain biological activity and is non-reactive with the subject's immune system. Examples include, but are not limited to, any of the standard pharmaceutical formulations such as a phosphate buffered saline solution, water, emulsions such as oil/water emulsion, and various types of wetting agents/surfactants and excipients. Preferred diluents for aerosol or parenteral administration are phosphate buffered saline or normal (0.9%) saline and water for injection.

Compositions comprising such formulations are formulated by well known conventional methods.

As used herein, "a" or "an" means at least one, unless clearly indicated otherwise.

Neural Progenitor Cells

The invention provides neural progenitor cells (NPC) that can be maintained indefinitely in culture and are multipotent. The NPC of the invention are capable of generating neurons (e.g., MAP2, neuron specific enolase or neurofilament positive cells) and glia (e.g., GFAP or galactocerebroside positive cells). NPC of the invention can be maintained in cell culture, typically as a suspension culture, for at least one year. NPC can be prepared from mesencephalon and/or telencephalon of fetal brain, as described in embodiment 3 below. Typically, the tissue is dissected in a general purpose serum-free medium, such as Hank's Balanced Salt Solution (HBSS) with 0.25 micrograms/ml of Fungizone and 10 micrograms/ml of Gentamicin, under sterile conditions.

Pluripotent Stem Cells

The invention provides pluripotent stem cells (PSC) that can be maintained indefinitely in culture, and that stain positively for the stem cell marker Oct4. The PSC of the invention coexpress Oct4 and nestin, indicating that these cells are capable of generating neurons and glia. PSC of the invention can be maintained in cell culture, typically as a suspension culture. The PSC of the invention can be used in all the ways described herein for NPC. The Oct4-positive status of these cells indicates that they are capable of many additional uses beyond the neural environment. The pluripotent nature of these cells make them attractive for placement in a variety of tissue environments, wherein local cytokines (natural and/or exogenously supplied) and other signals induce appropriate differentiation and migration. In the description of methods that follows, it is understood that NPC refers to NPC and/or PSC.

Media and Methods for Cell Culture

The structure and function of NPC in culture is subject to manipulation via the culture surface. For general purposes, the cell culture requires a low calcium basal medium (e.g., Ca⁺⁺ free EMEM supplemented with calcium chloride), typically a B27 or equivalent supplement, and growth factors (e.g., EGF, FGF, TGFα). Optional ingredients include L-glutamine and LIF, which promote growth of neurons.

Embodiment 3 provides a detailed description of the optimization of culture media for expansion and for differentiation of NPC. NPC are typically grown in suspension cultures. Initial plating of primary cells was optimal at 5,000 to 50,000 cells/cm². Cerebral cortical NSCs were purified and cultured in T75 culture flasks (Coming, USA) at a density of 50 000 cells/cm² in the culture medium described above and maintained at 37 °C in a humidified atmosphere of 95% air/5% CO₂. After 5 days of culture, suspended cells underwent cell division and proliferating cells formed neurospheres. Subsequently, adherent cells were discarded and neurospheres were collected by centrifugation, mechanically dissociated and replated as single cells in a new T75 culture flask at a density of 50 000 cells/cm₂ in the fresh culture medium. These single cells proliferated and grew into new spheres in the subsequent 5 days. The procedure of subculture was repeated again, and then cells were collected to seed onto glass coverslips modified with different chemical groups and cultured in the same culture medium as described above. The medium was changed after culture for 24 h and 72 h. After co-cultured at defined time, the samples were removed from the culture medium and washed three times in PBS before fixed in 4% formaldehyde for 30 min.

The NPC of the invention can be used in therapeutic and diagnostic applications, as well as for drug screening and genetic manipulation. The NPC and/or culture media of the invention can be provided in kit form, optionally including containers and/or syringes and other materials, rendering them ready for use in any of these applications.

Therapeutic Use of NPC

The NPC of the invention can be implanted into the central nervous system (CNS) of a host using conventional techniques. Neural transplantation or "grafting" involves transplantation of cells into the parenchyma, into the ventricular cavities or subdurally onto the surface of a host brain. Conditions for successful transplantation include: 1) viability of the implanted cells; 2) formation of appropriate connections and/or appropriate phenotypic expression; and 3) minimum amount of pathological reaction at the site of transplantation.

Therapeutic use of NPC can be applied to degenerative, demyelinating, excitotoxic, neuropathic and traumatic conditions of the CNS. Examples of conditions that can be treated via NPC grafts include, but are not limited to, Parkinson's disease (PD), Huntington's disease (HD), Alzheimer's disease (AD), multiple sclerosis (MS), amyotrophic lateral sclerosis (ALS), epilepsy, stroke, ischemia and other CNS trauma.

Methods for transplanting various neural tissues into host brains have been established. These procedures include intraparenchymal transplantation, i.e. within the host brain (as compared to outside the brain or extraparenchymal transplantation), achieved by injection or deposition of tissue within the host brain so as to be opposed to the brain parenchyma at the time of transplantation.

The procedure for intraparenchymal transplantation involves injecting the donor cells within the host brain parenchyma stereotactically. This is of importance if it is required that the graft become an integral part of the host brain and to survive for the life of the host. Typically, intraparenchymal transplantation involves pre-differentiation of the cells. Differentiation of the cells, however, limits their ability to migrate and form connections. Intraparenchymal transplantation of pre-differentiated cells is typically preferred when the objective is to achieve neurochemical production at the site of implantation.

Alternatively, the graft may be placed in a ventricle, e.g. a cerebral ventricle or subdurally, i.e. on the surface of the host brain where it is separated from the host brain parenchyma. For subdural grafting, the cells may be injected around the surface of the brain. In some embodiments, the NPC are injected intravenously. NPC introduced intraventricularly or intravenously will migrate to the appropriate region on the host brain. Intraventricular (or intravenous) transplantation is preferred when the objective is restoration of circuitry and function.

Injections into selected regions of the host brain may be made by drilling a hole and piercing the dura to permit the needle of a microsyringe to be inserted. The microsyringe is preferably mounted in a stereotaxic frame and three dimensional stereotaxic coordinates are selected for placing the needle into the desired location of the brain or spinal cord. For grafting, the cell suspension is drawn up into the syringe and administered to anesthetized graft recipients. Multiple injections may be made using this procedure. Examples of CNS sites into which the NPC may be introduced include the putamen, nucleus basalis, hippocampus cortex, striatum or caudate regions of the brain, as well as the spinal cord.

The cellular suspension procedure permits grafting of NPC to any predetermined site in the brain or spinal cord, is relatively non-traumatic, allows multiple grafting simultaneously in several different sites or the same site using the same cell suspension, and permits mixtures of cells having different characteristics. Multiple grafts may consist of a mixture of cell types, and/or a mixture of transgenes inserted into the cells. Preferably from approximately 10⁴ to approximately 10⁸ cells are introduced per graft. Optionally, the NPC can be grafted as clusters of undifferentiated cells. Alternatively, the NPC can be induced to differentiate prior to implantation.

For transplantation into cavities, which may be preferred for spinal cord grafting, tissue is removed from regions close to the external surface of the CNS to form a transplantation cavity, for example by removing glial scar overlying the spinal cord and stopping bleeding with a material such a gelfoam. Suction may be used to create the cavity. The stem cell suspension is then placed in the cavity.

Grafting of NPC into a traumatized brain will require different procedures. For example, the site of injury must be cleaned and bleeding stopped before attempting to graft. In addition, the donor cells should possess sufficient growth potential to fill any lesion or cavity in the host brain to prevent isolation of the graft in the pathological environment of the traumatized brain.

Genetically Modified NPC

The present invention also can use genetically modifying NPC for grafting into a target tissue site. The methods of the invention also contemplate the use of grafting of transgenic NPC in combination with other therapeutic procedures to treat disease or trauma in the CNS or other target tissue. Thus, genetically modified NPC and/or PSC of the invention may be co-grafted with other cells, both genetically modified and non-genetically modified cells, which exert beneficial effects on cells in the CNS. The genetically modified cells may thus serve to support the survival and function of the co-grafted, non-genetically modified cells.

Moreover, the genetically modified cells of the invention may be co-administered with therapeutic agents useful in treating defects, trauma or diseases of the CNS (or other target tissue), such as growth factors, e.g. nerve growth factor (NGF), gangliosides, antibiotics, neurotransmitters, neuropeptides, toxins, neurite promoting molecules, and anti-metabolites and precursors of these molecules, such as the precursor of dopamine, L-dopa.

Vectors carrying functional gene inserts (transgenes) can be used to modify NPC and/or PSC to produce molecules that are capable of directly or indirectly affecting cells in the CNS to repair damage sustained by the cells from defects, disease or trauma. In one embodiment, for treating defects, disease or damage of cells in the CNS, NPC are modified by introduction of a retroviral vector containing a transgene or transgenes, for example a gene encoding nerve growth factor (NGF) protein. The genetically modified NPC are grafted into the central nervous system, for example the brain, to treat defects, disease such as Alzheimer's or Parkinson's, or injury from physical trauma, by restoration or recovery of function in the injured neurons as a result of production of the expressed transgene product(s) from the genetically modified NPC. The NPC may also be used to introduce a transgene product or products into the CNS that enhance the production of endogenous molecules that have ameliorative effects in vivo.

Those skilled in the art will appreciate a variety of vectors, both viral and non-viral, that can be used to introduce the transgene into the NPC and/or PSC. Transgene delivery can be accomplished via well-known techniques, including direct DNA transfection, such as by electroporation, lipofection, calcium phosphate transfection, and DEAE-dextran. Viral delivery systems include, for example, retroviral vectors, lentiviral vectors, adenovirus and adeno-associated virus.

The nucleic acid of the transgene can be prepared by recombinant methods or synthesized using conventional techniques. The transgene may include one or more full-length genes or portions of genes. The polypeptides encoded by transgenes for use in the invention include, but are not limited to, growth factors, growth factor receptors, neurotransmitters, neuropeptides, enzymes, gangliosides, antibiotics, toxins, neurite promoting molecules, anti-metabolites and precursors of these molecules. In particular, transgenes for insertion into NPC include, but are not limited to, tyrosine hydroxylase, tryptophan hydroxylase, ChAT, serotonin, GABA-decarboxylase, Dopa decarboxylase (AADC), enkephalin, amphiregulin, EGF, TGF (α or (β), NGF, PDGF, IGF, ciliary neuronal trophic factor (CNTF), brain derived neurotrophic factor (BDNF), neurotrophin (NT)-3, NT-4, and basic fibroblast growth factor (bFGF).

In general, polypeptides (including fusion proteins) and polynucleotides as described herein are isolated. An "isolated" polypeptide or polynucleotide is one that is removed from its original environment. For example, a naturally occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

Treatment includes prophylaxis and therapy. Prophylaxis or therapy can be accomplished by a single direct injection at a single time point or multiple time points to a single or multiple sites. Administration can also be nearly simultaneous to multiple sites. Patients or subjects include mammals. The subject is preferably a human.

Administration and Dosage

The compositions are administered in any suitable manner, often with pharmaceutically acceptable formulations. Suitable methods of administering cells in the context of the present invention to a subject are available, and, more than one route can be used to administer a particular cell composition, a particular route can often provide a more immediate and more effective reaction than another route.

The dose administered to a patient, in the context of the present invention, should be sufficient to affect a beneficial therapeutic response in the patient over time, or to inhibit disease progression. Thus, the composition is administered to a subject in an amount sufficient to elicit an effective immune response to the specific antigens and/or to alleviate, reduce, cure or at least partially arrest symptoms and/or complications from the disease or condition. An amount adequate to accomplish this is defined as a "therapeutically effective dose."

Routes and frequency of administration of the therapeutic compositions disclosed herein, as well as dosage, will vary from individual to individual, and may be readily established using standard techniques. Typically, the pharmaceutical compositions are administered by injection. Preferably, between 1 and 10 doses may be administered over a 52 week period. Alternate protocols may be appropriate for individual patients.

A suitable dose is an amount of a compound that, when administered as described above, is capable of promoting a therapeutic response, and is at least a 5-90% improvement relative to the untreated level. In general, an appropriate dosage and treatment regimen provides the material in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

Pharmaceutical Compositions

The invention provides pharmaceutical compositions comprising NPC and/or PSC and, optionally, a physiologically acceptable carrier. Pharmaceutical compositions within the scope of the present invention may also contain other compounds that may be biologically active or inactive. For example, one or more biological response modifiers may be present, either incorporated into a fusion polypeptide or as a separate compound, within the composition.

While any suitable formulation known to those of ordinary skill in the art may be employed in the pharmaceutical compositions of this invention, the type of formulation will vary depending on the mode of administration. Compositions of the present invention may be formulated for any appropriate manner of administration, including for example, intracranial, intraventricular or subdural administration. Biodegradable microspheres (e.g., polylactate(PLA), polyglycolate(PGA), polymers of PLA and PGAs, Polycaprolactone and polydioxanes, and polymers of them) may also be employed as carriers for the pharmaceutical compositions of this invention. Suitable biodegradable microspheres are well known in the field such as natural polymers, such as alginate, collagen, gelation, chondrotin sulfate, heparin, hyluronic acid, etc. Such compositions may also comprise buffers (e.g., neutral buffered saline or phosphate buffered saline), carbohydrates (e.g., glucose, mannose, cellulose, sucrose or dextrans), mannitol, proteins, polypeptides or amino acids such as glycine, antioxidants, chelating agents such as EDTA or glutathione, adjuvants (e.g., aluminum hydroxide) and/or preservatives.

Detailed description of this invention is given below in conjunction with specific embodiments. Preferably, the culture medium is serum-free and free of human and animal products. The medium can further comprise DMEM/F12 with 0.2% to 3% B27, 0.5-50 ng/mL EGF, 0.2-30 ng/mL bFGF and 0.5-200 UI/mL penicillin and 0.5-300 µg/mL streptomycin. Typically, the growth factors, EGF, bFGF, LIF and TGFα, are recombinant growth factors.

Neural stem cell culture was prepared from pregnant Sprague Dawley rat embryos on day 13-15. Briefly, embryonic rat cerebral cortices were dissected, cut into small pieces and mechanically triturated in cold Hank's balanced salt solution. The NPC are derived from Sprague Dawley rat embryo cortical area and the culture methods. The NPC cultured in accordance with the invention have a doubling rate of less than 10 days, typically about 5 days.

The invention further provides a method of propagating neural progenitor cells, comprising culturing primary rat embryo brain cortical NPCs and NSCs in a culture surface and medium of the invention. In one embodiment (example 1), the OH chemical group was exhibited on the culture surface so that it suppress the differentiation of NPCs and NSCs.

The invention additionally provides a method of propagating pluripotent stem cells (PSC). The method comprises culturing primary rat embryo brain cortical tissue in a culture surface which exhibit ―NH₂ in this invention. In another embodiment (example 2), the NH₂ chemical group was presented on the culture surface so that it promotes the differentiation of NPCs and NSCs. The cultures can be monitored for the expression of Oct4 and Nestin, stem cell markers whose expression have been shown to increase in prevalence of the desired cell population among cells cultured by the method of the invention over a period of months.

**Embodiment 1: Preparation of ―OH group on the culture surfaces**

Preparation of model surfaces on glass coverslips Glass coverslips (10 mm diameter) were dipped into 1% sodium dodecyl sulfate (Sigma, USA) solution for 30 min followed by 0.1 M hydrochloric acid solution for 30 min. All the coverslips were then rinsed with deionized water and stored in deionized water prior to introduction of different functional groups on the surface.

To introduce -OH group, clean coverslips were dipped into Piranha solution freshly prepared from concentrated H₂SO₄ and 30% H₂O₂ at 80 °C for 30 min, then rinsed with deionized water and dried in nitrogen. Finally, these glass coverslips were placed in 48-well tissue culture polystyrene plates (Coming, USA), sterilized with 70% alcohol overnight and then rinsed extensively with sterilized phosphate-buffered saline (PBS). Finally, these glass coverslips were placed in 48 well tissue culture polystyrene plates (Coming, USA) for 15 s, and rinsed with toluene and ethanol, dried in nitrogen.

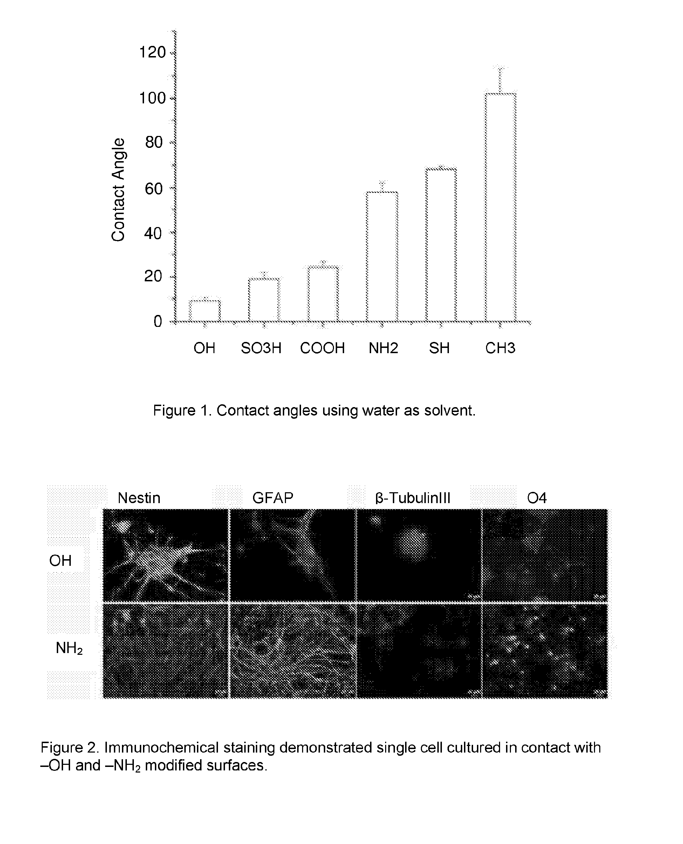

Water contact angles were measured on these -OH modified surfaces and the surfaces were having contact angles around 15° as shown in Figure 1.

Embodiment 2: Preparation of ―NH₂ group on the culture surfaces

To introduce ―NH₂ group, the -OH coverslips as shown in example 1 were dipped into 1% (3-aminopropyl)-triethoxy-silane (sigma, USA) or (3-mercaptogropyl)-trimethoxysilane (Sigma, USA) acetone solution, then 1mL water refluxing for 30 min. After refluxing, the coverslips were rinsed in deionized water and ethanol and then dried in nitrogen. Finally, these glass coverslips were placed in 48 well tissue culture polystyrene plates (Corning, USA) for 15 s, and rinsed with toluene and ethanol, dried in nitrogen.
The ―NH₂ modified surfaces were having contact angles around 60° as shown in Figure 1.

Embodiment 3: Culture of NPCs and NSCs
Neural stem cell culture was prepared from pregnant Sprague Dawley rat embryos on day 14―15 according to a standard protocol with some modification. Briefly, embryonic rat cerebral cortices were dissected, cut into small pieces and mechanically triturated in cold Hank's balanced salt solution. The dissociated cells were collected by centrifugation and resuspended in a serum-free medium containing DMEM-F12, 2% B27 supplement, 20 ng/ml EGF, 12.5 ng/ml bFGF and the following antibiotics, 100 UI/ml penicillin and 100 mg/ml streptomycin. The number of live cells was counted by trypan blue exclusion assay in a hemocytometer. Cerebral cortical NSCs were purified and cultured in T75 culture flasks (Coming, USA) at a density of 50,000 cells/cm² in the culture medium described above and maintained at 37 °C in a humidified atmosphere of 95% air/5% CO₂. After 5 days of culture, suspended cells underwent cell division and proliferating cells formed neurospheres. Subsequently, adherent cells were discarded and neurospheres were collected by centrifugation, mechanically dissociated and re-plated as single cells in a new T75 culture flask at density of 50,000 cells/cm² in the fresh culture medium.
These single cells proliferated and grew into new spheres in the subsequent 5 days. Both neurosphere-forming cells and single neural cell were examined for cell attachment, proliferation and differentiation. The procedure of subculture was repeated again, and then cells were collected to seed onto glass coverslips modified with OH (hydroxyl) chemical groups and NH₂ groups, and cultured in the same culture medium as described above. The medium was changed after culture for 24 h and 72 h. After co-cultured at defined time, the samples were removed from the culture medium and washed three times in PBS before fixed in 4% formaldehyde for 30 min.

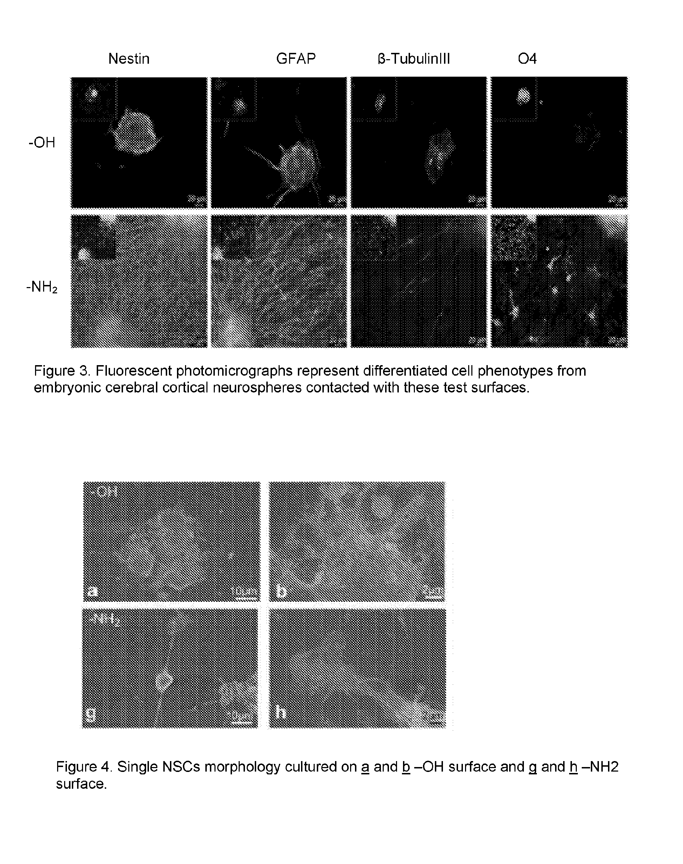

**Embodiment 4: Immunocytochemistry**
Fixed cells were incubated in PBS containing 0.3% triton-X-100 and 10% goat serum for 2h at room temperature and then incubated with primary antibodies at 4°C overnight. The primary antibodies and their dilution used in this study were mouse anti-nestin monoclonal IgG (1:400; Chemicon, USA), mouse anti-β-TubulinIII monoclonal IgG (1:800; Sigma, USA), mouse antiglial fibrillary acidic protein monoclonal IgG (anti-GFAP, 1:400; Chemicon, USA) and mouse anti-04 monoclonal IgM (1µg/ml; Sigma, USA). FITC-conjmicrogramsated secondary antibodies were used to visualize the signal by reacting with cells for 30 min at room temperature. The secondary antibodies and their dilution were FITC-conjmicrogramsated goat anti-mouse IgG (1:200; Chemicon, USA) and FITC-conjmicrogramsated goat anti-mouse IgM (1:128; Sigma, USA). Cells were also counterstained with 4,6-diamidino-2-phenylindole (DAPI; Roche,Germany). These immunostained cells were visualized by indirect fluorescence under the fluorescent microscope (Leica, Germany).
As shown in Figure 2, on OH surfaces at single cell level, cell clusters with extending processes outgrowth were identified as neural stem cells (anti-nestin immunoreactive) or astrocytes (anti-GFAP immunoreactive). It demonstrated that OH surfaces did not support the differentiation of single neural stem cells into neuron.
As shown in Figure 3, on OH surfaces at neurospheres level, the neuronal marker cells (04-immunoreactive cells) were not found in either neurosphere itself or migrating cells, indicating minimal cell differentiation.
As shown in Figure 2, on ―NH₂ surfaces at single cell level, most of single cells exhibited 04, GFAP and Tubulin-III positive, indicating that single cells have the ability to differentiate into three cell phenotypes, that is, astrocytes, neurons and oligodentrocytes.
As shown in Figure 3, on ―NH₂ surfaces at neurosphere level, -NH₂ tended to have a promotion effect on neuronal differentiation on NSCs and an inhibitory effect on the differentiation into oligodendrocytes.

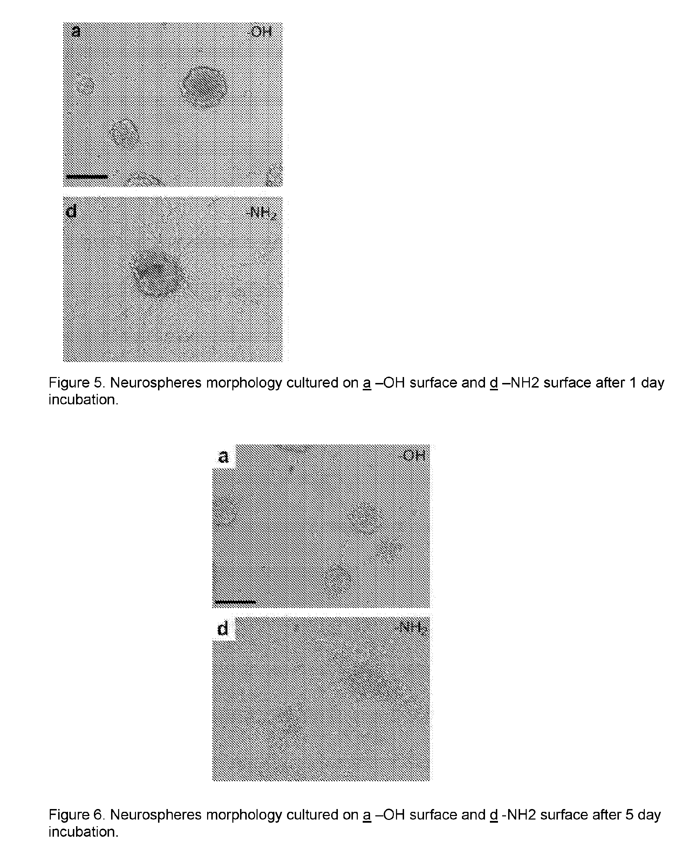

**Embodiment 5: Scanning electron microscopy (SEM)**
For samples observed by SEM, fixed samples were dehydrated through exposure to a gradient of alcohol and air-dried in a fume hood. After sputter-coated with gold, samples were observed using SEM (LEO-1530).
SEM revealed that stem cell adhered to -OH modified culture surfaces by exhibiting flattened cell morphology as shown in Figure 4a and 4b (single NSC). On NH₂ surfaces, both Figure 4b and 4h exhibited round and extended processes.
Migration behaviors of NSCs on OH modified surfaces were shown in Figure 5 for neurospheres in culture for 1 day. Figure 6 shows that neurosphers in culture for 5 days. There was the least degree of migration for NSCs on -OH modified culture surfaces which showed promise for -OH modified surfaces as a "storage" surface for NSC, whereas ―NH₂ surfaces can grow NSCs so that NSCs kept round and long processes. Cell migration was greater on ―NH₂ surfaces compared to other test substrates.

**Embodiment 6: 3-(4,5-Dimethylthiazol-2-yl)-diphenyl tetrazolium bromide (MTT) assay**
The cell viability was determined by the MTT (Sigma, USA) colorimetric assay. At the indicated time points, the cultures were added 50 microliter of MTT solution( 5 mg/mL in PBS) and incubated at 37 °C for 4 hours. Then, the culture medium was removed and rinsed in PBS for three times. The formazen reaction products were dissolved in dimethysulfoxide for 20 min. The optical density of the formazan solution was read on an ELISA plate reader at 490 nm.

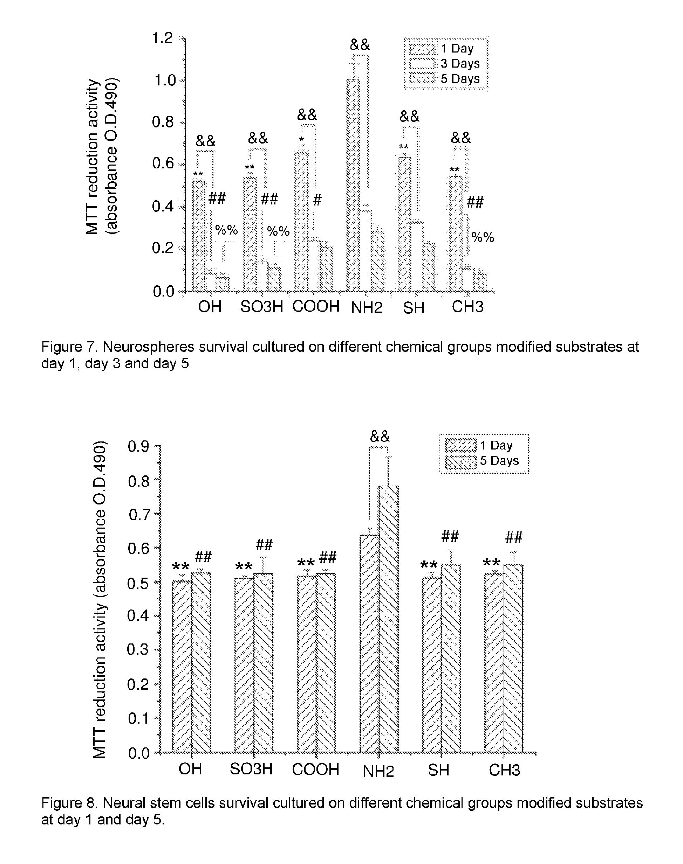

Figure 7 demonstrated for neurospheres, most number of cells adhere to ―NH₂ surfaces and least number of cells seeded on -OH surfaces at day 1. At day 5, there were more viable cells on NH₂ surfaces than on OH surfaces.
Figure 8 demonstrated for single NSC, -NH₂ surfaces adhered most number of viable cells at day 1 than -OH surfaces, and NH₂ surfaces support cell growth at day 5 better than -OH surfaces.

**EMBODIMENT 7: Focal adhesion staining and Actin staining of NSCs, NPCs and adipose stem cells on ―OH and NH₂ modified surfaces.**

### Focal adhesion stain

Cultured cells were fixed with 4% paraformaldehyde in PBS for 15-20 minutes at room temperature. The cells were washed twice with 1x wash buffer (0.05% Tween-20 in PBS). The cells were permeabilized with 0.1% Triton X-100 in 1x PBS for 1-5 minutes at room temperature. The cells were further washed twice with 1x wash buffer. A blocking solution (1%BSA) was applied for 30 minutes at room temperature. Primary antibody (Anti-Vinculin) was applied and incubated for 1 hour at room temperature. Then a washing step was repeated for three times (5-10 minutes each) with 1x wash buffer. A secondary antibody (FITC-conjugated, for example) was applied and incubated for 30-60 minutes at room temperature. For double labeling TRITC-conjugated Phalloidin can be incubated simultaneously with the secondary antibody for 30-60 minutes at room temperature. The culture was then washed three times (5-10 minutes each) with 1x wash buffer. Then the nuclei counterstaining can be performed by incubating cells with DAPI for 1-5 minutes at room temperature, followed by washing cells three times (5-10 minutes each) with 1 x wash buffer.

### Actin stain

Actin staining was performed by washing the cells twice with prewarmed PBS. The cells were then fixed in 4% formaldehyde solution in PBS for10 minutes at room temperature. The cells were further washed two or more times with PBS. PBS was removed and put 0.1% TRITON® X-100 in PBS for 5 minutes. Repeated washing was done by washing two or more times with PBS/1% BSA. The rhodamine phalloidin solution was placed in well for 30 Min- 1 hours at room temperature. Two or more washing was done with PBS. The SYTOX® Green staining solution was placed in well for 10 Min at room temperature. Cells were then washed for two or more times with PBS.

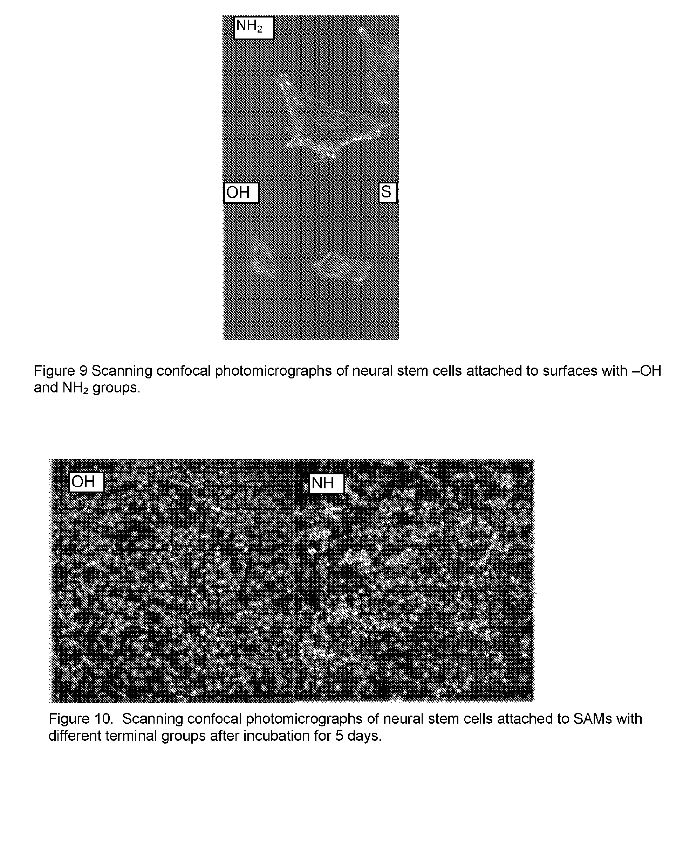

Focal adhesion staining results can be seen in Figure 9. The difference in the initial cell spreading were confirmed by the analysis of the focal adhesion complexes using vinculin (green), actin (red) and nuclei (blue) labeling as shown in the Fig.9 . Neural stem cells attached to NH₂ after incubation for 12 hours contained short linear focal adhesion plaques at the cell periphery and longitudinal actin stress fibres, with actin partly colocalized with vinculin in the focal adhesion plaques indicated by the yellow color. These cells were fully spread within 12 hours with many bundles of actin stress fibres anchored to the plasma membrane at sites of extended focal adhesion contacts.

In contrast, on OH modified surfaces, no focal adhesions were observed nor any formation of actin stress fibres, meanwhile the colocalization of actin and vinculin indicated by the yellow color were hardly found. Obviously, the formation of focal contacts was absent on OH modified surfaces.

The influence of different chemical groups on materials surfaces on cell attachment was investigated by the analysis of the distribution of cells using actin (red) and nulei (green) labeling as shown in the Fig.10. After incubation for 5 days and double labelling, NH₂ modified surfaces have seeded more NSCs and these neural stem cells were evenly distributed.

**EMBODIMENT 8: Culture of human-adipose stem cells**
Human adipose-derived stem cells (ASCs) were harvested from the subcutaneous adipose tissue of a female donor during liposuction. This procedure was carried out under her voluntary consent.
Isolation and culture of stem cells-Processed Lipoaspirates (PLA) and Mesenchymal stem cells (MSCs)
Human adipose tissue was obtained from elective liposuction procedures under local anesthesia. In this procedure, a hollow blunt-tipped cannula was introduced into the subcutaneous space through small (~1 cm) incisions. The cannula was attached to gentle suction and moved through the adipose compartment, mechanically disrupting the fat tissue. A solution of saline and the vasoconstrictor epinephrine was infused into the adipose compartment to minimize blood loss and contamination of the tissue by peripheral blood cells. The raw lipoaspirate (~300 cc) was processed according to established methodologies to obtain a stromal vascular fraction (SVF). To isolate the SVF, lipoaspirates were washed extensively with equal volumes of phosphate-buffered saline (PBS), and the ECM was digested at 37°C for 30 min with 0.075% collagenase. Enzyme activity was neutralized with Dulbecco's modified Eagle's medium (DMEM), containing 10% FBS and centrifuged at 1200 3 *g* for 10 min to obtain a high-density SVF pellet. The pellet was resuspended in 160 mM NH₄Cl and incubated at room temperature for 10 min to lyse contaminating red blood cells. The SVF was collected by centrifugation, as detailed above, filtered through a 100-*m*m nylon mesh to remove cellular debris and incubated overnight at 37°C/5% CO2 in control medium (DMEM, 10% FBS, 1% antibiotic/antimycotic solution). Following incubation, the plates were washed extensively with PBS to remove residual nonadherent red blood cells. The resulting cell population was termed a processed lipoaspirate (PLA), to distinguish it from the SVF obtained from excised adipose tissue. PLA cells were maintained at 37°C/5% CO₂ in noninductive control medium. Cells did not require specific FBS sera lots for expansion and differentiation. For immunofluorescence studies, a population of MSCs was obtained from human bone marrow aspirates according to the protocol and maintained in control medium. To prevent spontaneous differentiation, cells were maintained at subconfluent levels.

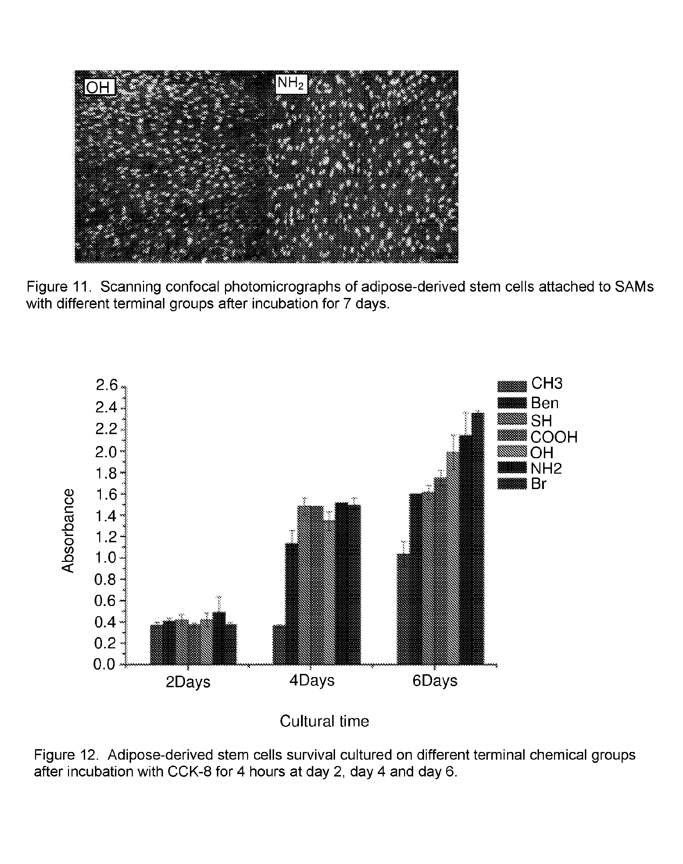

**EMBODIMENT 9: Scanning confocal hotomicrographs of adipose-derived stem cells attached to chemical groups-modified surfaces after incubation for 4 hours.**
The influence of different terminating groups on cell attachment was investigated by the analysis of the distribution of cells using actin (red) and nulei (green) labeling as shown in the Fig.11. After incubation for 7 days and doublelabelling, adipose-derived stem cells clustered and the seeding density of cells was high on -OH and -NH₂ modified surfaces, and cells were evenly distributed.

EMBODIMENT 10: Attachment and proliferation of adipose-derived stem cells cultured on different chemical groups-modified surfaces.
CCK-8 assay
After incubating at 37°C and 5% CO₂ for 2, 4 and 6 days, the culture medium was replaced with 100 microliter medium containing CCK-8 (10µl each well). After cultured 4 h at 37°C and 5 % CO₂, 100 microliter solution of each sample was placed in a 96-well plate and the optical densities at 450 nm were measured with microplate reader (Bio-Rad, Model 680). Five samples for each group were tested in the experiment.
The optical absorbance of supernatants of adipose-derived stem cells after incubation with CCK-8 for 4 hours was used to determine the amount of adipose-derived stem cells after certain time periods (Figure 12). At day2, there are no significant differences among the adsorbance of the supernatants from different terminating groups. However, when it came to 4 days and 6 days, the absorbance of the supernatants from OH and NH₂ were significantly higher than other surfaces, which indicated that at day 4 and day 6, there were significant more adipose-derived stem cells on OH and NH₂ modified surfaces.

The following paragraphs contain statements of broad combinations of the inventive technical features herein disclosed:-
A. A stem cell culture comprising culturing primary human cells in a culture surface such that surface comprising an OH concentration of the surface is such that the surface contact angles ranging from 5°-35°.
B. A stem cell culture comprising culturing primary human cells in a culture surface such that surface comprising a NH₂ concentration of the surface is such that the surface contact angles ranging from 50°-79°.
C. The culture surfaces of paragraph A wherein the chemical functional group: OH concentration is such that the surface contact angles ranging from 10-20°.
D. The culture surfaces of paragraph B wherein the chemical functional group: NH₂ concentration is such that the surface contact angles ranging from 55-68°.
E. A cell culture comprising a cell culture surfaces of paragraph A and culture medium.
F. A cell culture comprising a cell culture surfaces of paragraph B and culture medium.
G. The cell culture of paragraph A, wherein the cells are derived from human brain, bone marrow, or human adipose tissue.
H. The cell culture of paragraph B, wherein the cells are derived from human brain, bone marrow, or human adipose tissue.
I. The cell culture of paragraph A, wherein the cells have a doubling rate of less than 12 days.
J. The cell culture of paragraph A, wherein the cells have a doubling rate of about 5 days.
K. The cell culture of paragraph B, wherein the cells have a doubling rate of about 5 days.
L. The cell culture of paragraph A, wherein the cells are obtained from fetal forebrain.
M. A method of propagating NPC and NSCs that are immunopositive for nestin and Oct4, comprising culturing primary human fetal brain tissue in a culture surface as in any one of paragraphs A to L.
N. A method of transplanting human NPC to a mammalian host, comprising:
   (a) obtaining a cell culture of any one of paragraphs A to L; and
   (b) transplanting the cell culture to the central nervous system (CNS) of the host, wherein the host has Parkinson's disease or epilepsy.
O. A method of propagating NPC and NSCs that are immunopositive for nestin and Oct4, comprising culturing primary human fetal brain tissue in a culture surface as in any one of claims paragraph B to L.
P. A method of transplanting human NPC to a mammalian host, comprising:
   (a) obtaining a cell culture of any one of paragraphs B to L; and
   (b) transplanting the cell culture to the central nervous system (CNS) of the host, wherein the host has central nervous disease.
Q. The method of paragraph H and I, wherein the cell culture is transplanted to a ventricle of the central nervous system.

## Claims

1. A stem cell culture comprising primary human cells in contact with a culture surface, the surface comprising an OH concentration such that the surface contact angle is in the range from 5° to 35°.

2. A stem cell culture comprising primary human cells in contact with a culture surface, the surface comprising a NH₂ concentration such that the surface contact angle is in the range from 50°-79°.

3. The culture of claim 1 wherein the chemical functional group OH concentration is such that the surface contact angle ranges from 10-20°.

4. The culture of claim 2 wherein the chemical functional group NH₂ concentration is such that the surface contact angle ranges from 55-68°.

5. A cell culture comprising a cell culture surface of claim 1 and culture medium.

6. A cell culture comprising a cell culture surface of claim 2 and culture medium.

7. The cell culture of claim 1 or claim 2, wherein the cells are derived from human brain, bone marrow, or human adipose tissue.

8. The cell culture of any one of claims 1 to 7, wherein the cells have a doubling rate of less than 12 days.

9. The cell culture of any one of claims 1 to 7, wherein the cells have a doubling rate of about 5 days.

10. The cell culture of any one of claims 1 to 9, wherein the cells are obtained from fetal forebrain.

11. A method of propagating NPC and NSCs that are immunopositive for nestin and Oct4, the method comprising culturing primary human fetal brain tissue on a culture surface according to any one of claims 1 to 4, wherein the method is preferably an *in vitro* method.

12. Human neural progenitor cells (NPC) for use in a method of treatment of a mammalian host, wherein the human NPC are obtained from a cell culture according to any one of claims 1 to 4, and preferably one of claims 1 or 3, and wherein the method of treatment comprises transplanting the NPC to the central nervous system (CNS) of the mammalian host, wherein the host has Parkinson's disease or epilepsy, the method preferably providing a treatment for said Parkinson's disease or epilepsy.

13. Human neural progenitor cells (NPC) for use in a method of treatment of a mammalian host, wherein the human NPC are obtained from a cell culture according to any one of claims 1 to 4, and preferably one of claims 2 or 4, and wherein the method of treatment comprises transplanting the NPC to the central nervous system (CNS) of the mammalian host, wherein the host has central nervous system disease, the method preferably providing a treatment for said central nervous system disease.

14. Human neural progenitor cells (NPC) for use in a method of treatment according to claim 12 or claim 13, wherein the NPC are derived from human brain, bone marrow, or human adipose tissue, and wherein the cells have a doubling rate in said cell culture of less than 12 days, or optionally of about 5 days, wherein said transplantation is to a ventricle of the central nervous system.
